(19) Europäisches Patentamt
European Patent Office
Office européen des brevets

(11) Publication number: 0 191 520 B1

(12) # EUROPEAN PATENT SPECIFICATION

(45) Date of publication of patent specification: 21.06.89

(51) Int. Cl.$^4$: A 61 K 31/415, C 07 D 231/56

(21) Application number: 86200115.3

(22) Date of filing: 29.01.86

(54) (1-phenylmethyl-5-hydroxy-1H-indazol-3-yl)-oxyacetic acid and salts thereof for use as a medicament, and pharmaceutical compositions containing them.

(30) Priority: 12.02.85 IT 4767185
25.09.85 IT 2227585

(43) Date of publication of application:
20.08.86 Bulletin 86/34

(45) Publication of the grant of the patent:
21.06.89 Bulletin 89/25

(84) Designated Contracting States:
AT BE CH DE FR GB IT LI LU NL SE

(56) References cited:
FR-M- 7 174
US-A-4 451 477

M. GIANNANGELI et al, Boll. Chim. Farm. 121 (1982), pp. 465-474

THE LANCET, vol. I, April 1982, London: M. Testa et al. 2pilot Study of Bendazac for Treatment of Cataract" pages 849-850

(73) Proprietor: AZIENDE CHIMICHE RIUNITE ANGELINI FRANCESCO A.C.R.A.F. S.p.a.
Viale Amelia, 70
I-00181 Roma (IT)

(72) Inventor: Silvestrini, Bruno
Via Michelangelo Schipa, 15
I-00181 Roma (IT)

(74) Representative: Marchi, Massimo et al
c/o Marchi & Mittler s.r.l. Viale Lombardia 20
I-20131 Milano (IT)

EP 0 191 520 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the pharmaceutical use of (1-phenylmethyl-5-hydroxy-1H-indazol-3-yl)-oxy-acetic acid and of pharmaceutically acceptable salts thereof with organic and inorganic bases as well as to pharmaceutical compositions containing them.

The cataract is an opacification of the crystalline lens, a body in the form of a lens interposed between the anterior chamber and the vitreous body of the eye, which causes the loss of its transparency.

Until recently the only possible cure for cataract was considered the surgical removal of the crystalline lens and its replacement with an artificial lens.

Although there have appeared in late years several sporadic references to the use of various drugs in the therapy of the cataract in man only two drugs are actually used: pirenoxine and bendazac lysinate.

Pirenoxine is a pyridophenoxazine derivative and is only administered topically while bendazac lysinate is an indazole derivative which is administered systemically and has the following formula:

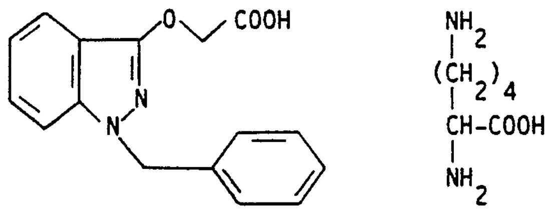

I

The anticataract properties of (I) were described rather recently (M. Testa, G. Juliano, B. Silvestrini, The Lancet, 1, April 10, 1982, 849—850; U.S. Patent 4,451,477).

Since the mechanism of action of this drug is still a matter of discussion and scientific speculation, among the various possible factors which bring about its action, at least on experimental laboratory cataracts, a partial contribution of a metabolite has been suggested. (G. Iuliano, and D. Santino, Archivio Siciliano di Medicina e Chirurgia, Vol. XXII, No. 6 December 1981; M. Testa, Italian Patent Application No. 20454 A/83 filed on March 31, 1983).

One of the metabolites of bendazac was isolated and described by M. Giannangeli et al. (Bolletino Chimico Farmaceutico Vol. *121,* 465, 1982). This compound is (5-hydroxy-1-benzyl)-indazolyl-3-oxyacetic acid and has the following structural formula:

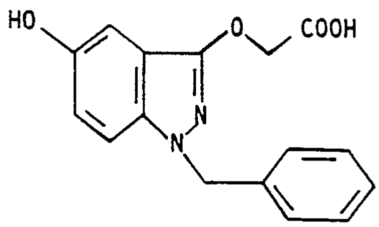

II

but no pharmacological property has yet been reported for it nor have its salts with pharmaceutically acceptable bases been described except for sodium salt which was only described as an intermediate in the preparation of II.

In the meantime however search by M. Testa into possible active metabolites not only of bendazac but of other drugs capable of exhibiting a therapeutic action on the cataract only systemically and not topically has given important results.

A very active fraction has been identified in the sputum of patients treated with the above drugs.

Said fraction consists of neutral or basic compounds soluble in organic solvents (M. Testa, Italian Patent Application No. 21398 A/83 filed on June 1, 1983).

According to this document the administration of drugs which are antiinflammatory in nature can give rise to the accumulation in the sputum of some patients of neutral or basic compounds which, apart from their endogenous or metabolic nature, show a potent therapeutic action on the cataract.

It has now been unexpectedly found that (5-hydroxy-1-benzyl)-indazolyl-3-oxyacetic acid (II) even though it has a purely acid nature and is thus different from the anticataract agents described in Italian Patent Application No. 21398 A/83 is endowed with anticataract activity also when administered topically. The same activity is also shown by the salts of Compound (II) with pharmaceutically acceptable inorganic and organic bases.

An object of this invention is therefore the pharmaceutical use of Compound (II) and of pharmaceutically acceptable addition salts thereof with a base; owing to their protective action on protein denaturation they may be used in all those afflictions in which such a type of phenomenon occurs.

Examples of said afflictions are the inflammatory diseases and cataract.

The Compounds of formulas I and II were tested under experimental conditions designed to show the protective effect on protein denaturation from UV rays according to the method of Silvestrini et al. (Int. J. Tiss. Reac. 5, 217—225, 1983) which comprises the measurement of the protective action of the compound under examination on the denaturation of the albumin of beef serum submitted to UV rays for 30 minutes.

The results obtained are shown below.

COMPOUND I

| Molar Conc. | % Inhibition | Mean |
|---|---|---|
| .002 | 53—60—68—64—62 | 61% |
| .001 | 30—40—48—45—43—45—54—35 | 42% |
| .0005 | 4—24—28—24—32—12 | 21% |
| .00025 | 7—18—13—14—4—15 | 12% |

COMPOUND II

| Molar Conc. | % Inhibition | Mean |
|---|---|---|
| .0002 | 85—100 | 92% |
| .0001 | 63—68—100 | 77% |
| .00005 | 8—13 | 10% |

These results indicate that Compound II in this test is at least 10 times as active as Compound I.

Preliminary tests on man confirmed the action of Compound II in cataract therapy.

Two groups of 10 patients afflicted by bilateral senile cataract with cortical or subcapsular or cortinuclear localization, in the initial evolutive stage, without fundus alterations and with a distance visus from 5 to 9 tenths (after correction of any refraction error) were treated for three months with Compound II orally (100 mg tablet; 1 tablet, three times a day) and topically (0.1% collyrium; 1 drop in the conjuctival sac of each three times a day) respectively.

The effect of the treatment was evaluated by monitoring the variations in the visus, in the refraction error, and in lenticular opacity (by biomicroscopy with a slit lamp and by ophthalmoscopy).

In the group treated orally (tablets) the mean visus value increased 16.79% and said improvement proved highly significant after statistical analysis ($t = 3.58$; $P < 0.01$), opacity decreased in 6 eyes, remained unchanged in 13 and increased only in 1 eye. According to overall clinical judgement, 7 patients improved (of which 4 greatly), 2 remained unchanged, and only one worsened slightly.

In the group treated topically (collyrium) the mean visus value increased 12.78% and said improvement proved significant after statistical analysis ($t = 2.54$; $P < 0.05$); opacity decreased in 7 eyes, remained unchanged in 11, and increased in 2.

According to overall clinical judgement, 4 patients improved, 5 remained unchanged and only one worsened.

Another object of this invention are the new salts of Compound II with pharmaceutically acceptable organic or inorganic bases. Examples of suitable inorganic bases are potassium and calcium hydroxide. Examples of suitable organic bases are methylamine, isopropylamine, hexylamine, dimethylamine, ethanolamine, 2-hydroxymethyl-2-amino-1,3-propanediol, glycine, alanine, valine, leucine, isoleucine, serine, threonine, aspartic acid, glutamic acid, arginine, lysine, cystine, cysteine, methionine, phenylalanine, and tyrosine.

Said salts may be prepared with conventional methods and in the case of liquid pharmaceutical compositions even at the time the pharmaceutical composition is prepared.

A further object of this invention are the pharmaceutical compositions containing Compound II or a pharmaceutically acceptable addition salt thereof with a base together with pharmaceutically acceptable carriers.

These compositions are preferably suitable for oral or topical administration and may be solid as for example tablets, capsules, powders, and granules; or liquid like for example solutions, suspensions or emulsions, or semiliquid like creams or ointments.

They may also be prepared in such a way as to allow delayed release of the active ingredient or ingredients.

In addition to the carriers they may also contain preservatives, stabilizers, wetting and emulsifying agents, salts for regulating the osmotic pressure, buffers, colouring and flavouring agents.

They may also contain other active ingredients and may be prepared by methods known to a person skilled in the art.

The following examples are given to illustrate the invention without limiting it in any way.

Example 1

Syrup
100 ml contain:

| | |
|---|---|
| sodium (1-phenylmethyl-5-hydroxy-1H-indazol-3-yl)-oxyacetate | 3.00 g |
| saccharose | 66.00 g |
| ethyl p-hydroxybenzoate | 0.15 g |
| sodium benzoate | 0.50 g |
| raspberry flavour | 0.50 g |
| distilled water q.s. to | 100.00 ml |

Example 2

Collyrium
100 ml contain:

| | |
|---|---|
| sodium (1-phenylmethyl-5-hydroxy-1H-indazol-3-yl)-oxyacetate | 0.250 g |
| hydroxypropyl-methyl-cellulose | 0.500 g |
| $H_2NaP_4.H_2O$ | 0.018 g |
| $NHa_2PO_4.12H_2O$ | 0.190 g |
| NaCl | 0.770 g |
| thimerosal | 0.001 g |
| water for injections q.s. to | 100.000 ml |

Example 3

Collyrium

| | |
|---|---|
| sodium (1-phenylmethyl-5-hydroxy-1H-indazol-3-yl)-oxyacetate | 0.500 g |
| hydroxy-propyl-methyl-cellulose | 0.500 g |
| $H_2NaP_4.H_2O$ | 0.018 g |
| $NHa_2PO_4.12H_2O$ | 0.190 g |
| NaCl | 0.740 g |
| thimerosal | 0.001 g |
| water for injections q.s. to | 100.000 ml |

Example 4

Ointment for ophthalmic use

| | |
|---|---|
| micronized (1-phenylmethyl-5-hydroxy-1H-indazol-3-yl)-oxyacetic acid | 0.5 g |
| soft yellow paraffin | 80.0 g |
| liquid paraffin q.s. to | 100.0 g |

## Claims

1. (1-phenylmethyl-5-hydroxy-1H-indazol-3-yl)-oxyacetic acid and pharmaceutically acceptable addition salts thereof with a base for use as a medicament.

2. (1-phenylmethyl-5-hydroxy-1H-indazol-3-yl)-oxyacetic acid and pharmaceutically acceptable addition salts thereof with a base for use as an anticataract agent.

3. A pharmaceutically acceptable addition salt of (1-phenylmethyl-5-hydroxy-1H-indazol-3-yl)-oxyacetic acid with a pharmaceutically acceptable organic or inorganic base except for sodium salt.

4. A pharmaceutical composition containing an effective amount of (1-phenylmethyl-5-hydroxy-1H-indazol-3-yl)-oxyacetic acid or of a pharmaceutically acceptable addition salt thereof with a base together with a pharmaceutically acceptable non-active ingredient.

## Patentansprüche

1. (1-Phenylmethyl-5-hydroxy-1H-indazol-3-yl)-oxyessigsäure und deren pharmazeutisch verträgliche Additionssalze mit einer Base zur Verwendung als Arzneimittel.

2. (1-Phenylmethyl-5-hydroxy-1H-indazol-3-yl)-oxyessigsäure und deren pharmazeutisch verträgliche Additionssalze mit einer Base zur Verwendung als Mittel gegen katarakt.

3. Pharmazeutisch verträgliches Additionssalz von (1-Phenylmethyl-5-hydroxy-1H-indazol-3-yl)-oxyessigsäure mit einer pharmazeutisch verträglichen organischen oder anorganischen Base mit Ausnahme des Natriumsalzes davon.

4. Pharmazeutisches Mittel, enthaltend eine wirkasame Menge (1-Phenylmethyl-5-hydroxy-1H-indazol-3-yl)-oxyessigsäure oder eines pharmazeutisch verträglichen Additionssalzes mit einer Base zusammen mit einem pharmazeutisch verträglichen nichtaktiven Bestandteil.

## Revendications

1. Acide (1-phénylméthyl-5-hydroxy-1H-indazol-3-yl)-oxyacétique et ses sels d'addition avec une base pharmaceutiquement acceptables à utiliser comme médicament.

2. Acide (1-phénylméthyl-5-hydroxy-1H-indazol-3-yl)-oxyacétique et ses sels d'addition avec une base pharmaceutiquement acceptables à utiliser comme agent anti-cataracte.

3. Un sel d'addition pharmaceutiquement acceptable de l'acide (1-phénylméthyl-5-hydroxy-1H-indazol-3-yl)oxyacétique avec une base organique ou inorganique pharmaceutiquement acceptable à l'exception du sel de sodium.

4. Une composition pharmaceutiquement contenant une quantité efficace d'acide (1-phénylméthyl-5-hydroxy-1H-indazol-3-yl)-oxyacétique ou d'un sel d'addition pharmaceutiquement acceptable de ce dernier avec une base ensemble avec un ingrédient non actif pharmaceutiquement acceptable.